# EUROPEAN PATENT APPLICATION

(11) **EP 3 242 124 A1**
(43) Date of publication of application: **08.11.2017**
(21) Application number: 16168207.5
(22) Date of filing: 03.05.2016
(51) Int. Cl.: G01N 21/85

(54) **DETECTION OF FOREIGN MATTER IN ANIMAL PRODUCTS**

(71) Applicant: TOMRA Sorting NV, 3001 Leuven (BE)
(72) Inventor: Boeykens, Steven, 3001 Leuven (BE); de Jonghe, Jan, 3001 Leuven (BE); Balthasar, Dirk, 56218 Mulheim-Karlich (DE)
(74) Representative: Tomkins & Co

(57) **Abstract**

An apparatus and method for detecting foreign matter in meat product is provided, the apparatus comprising: at least one detector arranged to detect foreign matter in falling meat product; and at least one spreader for increasing the spread of meat product prior to its falling.

## Description

### Field of the Invention

The present invention relates to the detection of foreign matter in animal products and in particular to the detection of foreign matter in streams of animal products or by-products in the food production industry.

### Background to the Invention

The detection of foreign matter (e.g. metal, glass, paper, plastics) in animal products and by-products is critical to ensure that such matter does not find its way into finished consumable food items. Existing techniques within the food industry provide for the detection of such matter in food streams using x-ray scanning. However, x-ray scanning is only suitable for the detection of objects or matter that are of a different density than the surrounding animal product. For meat products, such detection will not readily detect foreign matter such as plastics, wood or paper as such matter may be similar in density or less dense than the meat.

It is therefore desirable to provide an apparatus that improves the detection of a range of different types of foreign matter in animal products and by-products, and more particularly in streams of meat products common in the food production industry.

### Summary of the Invention

The present invention provides an apparatus for detecting foreign matter in meat product comprising: at least one detector arranged to detect foreign matter in falling meat product; and at least one spreader for increasing the spread of meat product prior to its falling.

This arrangement is advantageous as it provides for contactless investigation of meat products to detect foreign matter.

The apparatus may further comprise a conveyor, in which case there would be provided an apparatus for detecting foreign matter in meat product comprising a conveyor; at least one spreader for spreading meat product across the conveyor and a detector arranged to detect foreign matter in meat product falling from the conveyor.

The apparatus may further comprise a conveyor across which the meat product is spread by the spreader. The conveyor may be a belt conveyor.

The conveyor may comprise at least one inclined portion. The conveyor may comprise at least one declined portion. The conveyor may comprise multiple portions wherein at least two portions have different inclinations. Each inclination may be positive or negative.

Preferably the or each detector is arranged to scan the falling meat product with respect to a reference surface, in use the meat product falling between the detector and the reference surface. The or each detector may comprise an integrated light source or at least one external light source may be provided to illuminate the meat product.

The reference surface may comprise a stainless steel surface or any other suitable surface.

The apparatus may comprise a first detector arranged to scan the meat product from a first direction and a second detector arranged to scan the meat product from a second direction, different from the first. The two detectors may scan the meat product from opposite directions.

The or each spreader may comprise a plurality of channels, such that meat received at the spreader is distributed between the channels. The spreader may comprise a plurality of elongate separators defining the channels. Each channel may be further split into multiple channels at a least one point along its length.

The plurality of elongate separators may extend radially from a region on the surface of the spreader. The spreader may have a fan-shaped form.

The apparatus may comprise elongate separators of a first length for providing a first separation of the meat and elongate separators of a second shorter length for providing a second separation of the meat.

The apparatus may comprise alternating separators of the first length and the second length. Multiple spreaders may be provided. Where multiple spreaders are provided, they may be identical or employ different spreading means.

According to invention there is further provided a method for detecting foreign matter in meat product comprising: increasing the spread of the meat product; and dropping the spread meat product in front of at least one detector to detect foreign matter in the meat product when falling.

The spreader spreads the meat to prevent clumping of the meat and to reduce the thickness of the meat. This serves to singulate the meat product. Such spreading provides for a more uniformly thick layer of meat falling in front of the or each detector in which foreign matter is more readily detectable. In this manner, a more even distribution of meat is provided. Where a conveyor is provided, spreading across the conveyor as described does not require that the meat be spread across the entire width of the conveyor. Furthermore, arranging the detector to scan the meat product whilst the product is falling from the conveyor further provides for improved detection of foreign matter when compared to scanning the meat product whilst still on the conveyor.

At least one inclined portion facilitates the use of the apparatus with machines, for example meat grinders, with outlets positioned at a low height. An inclined portion provides a vertical component to the conveyance of the meat product. An inclined portion thus has the effect of conveying the meat up to a sufficient height such that the meat falls for a long enough period of time to be accurately scanned for foreign matter by the detector. At least one inclined portion of the conveyor may be inclined at an angle of about 10 degrees with respect to the horizontal.

The spreader may be provided on or above a conveyor, at any position along the length of the conveyor. The spreader may be located between consecutive portions of a multi-portion conveyor.

The detector may comprise a spectrometer. At least one beam from the spectrometer may be arranged to scan the meat product falling from the conveyor through a detection zone about the end of the conveyor. The wavelength of an illuminating beam from the spectrometer may be in the visible and/or near infra-red, NIR, region. Using a spectrometer to scan the falling meat provides for the detection of a much wider range of foreign matter than conventional techniques. Furthermore, spectroscopy can reveal matter not detectable by x-ray, for example paper or plastics. NIR spectroscopy is further effective for the detection of such materials. Other detection means and methods may alternatively be provided, such as but not limited to laser scattering, or camera-based image processing (hyperspectral camera or color-camera or infrared camera) systems.

The detector may be arranged to scan the meat product with respect to a reference surface, in use the meat product falling between the detector and the reference surface. The reference surface provides for enhanced accuracy of detection.

Stainless steel is an effective background reference against which to detect the presence of foreign matter. Furthermore, stainless steel is an accepted material for use in the food industry and can furthermore safely sustain a daily cleaning procedure. Alternative forms of reference surface may be provided.

Scanning via two detectors arranged to scan from different directions provides the advantage of enhanced detection of laminar type foreign matter, for example paper, which may be difficult to detect if scanned side-on by a single detector.

Rather than simply passing the meat onto the conveyor, the channels of the spreader have the effect of dividing the meat and thus distributing the meat more evenly across the width of the conveyor. The elongate separators have the effect of further guiding the meat for more even distribution on the conveyor. The spreader is adapted to receive a meat product stream having a first width and output a meat product stream having a second width greater than that of the first width. The spreader may be positioned beneath the meat product stream and adapted to spread meat product received into or onto the spreader from above.

The use of separators which radially extend from a region on the surface of the spreader is advantageous as meat arriving at the separator in bulky segments is spread or fanned out by the separators across the surface of the conveyor.

The spreader may thus provide two stages of meat separation. The first stage may be provided by the elongate separators of the first length and the second stage provided by the elongate separators of the second shorter length. Alternating separators of the first length and the second length is advantageous as the second shorter length separators further divide the meat product which has been initially spread by the first length separators. The arrangement of alternating shorter and longer separators further enhances the spreading effect. Other forms of spreader may be provided.

### Description of the Drawings

Figure 1 is a schematic representation of the apparatus according to one embodiment of the invention
Figure 2 is a representation of an apparatus for detecting foreign matter according to one embodiment of the invention.
Figure 3 is a side view representation of the apparatus of Figure 2.
Figure 4 is a top view representation of the apparatus of Figure 2.
Figure 5 is a schematic representation of the apparatus according a further embodiment of the invention.

### Detailed Description of the Drawings

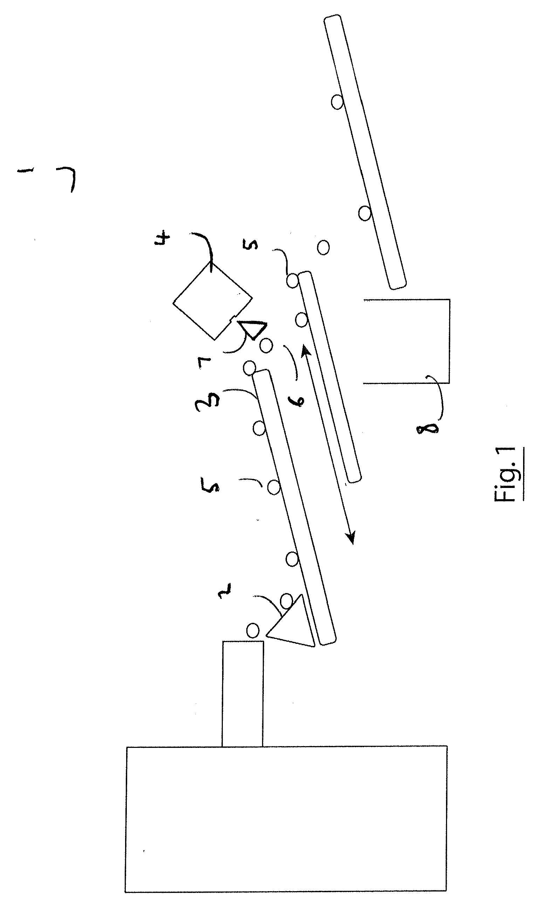

The present invention will now be described with reference to the accompanying drawings. Figure 1 is a schematic representation of one embodiment of the invention in use. The apparatus 1 comprises a spreader 2, a conveyor 3, and a detector 4 arranged to detect foreign matter in the meat product 5 falling from the conveyor 3. The region about the conveyor through which the meat falls is termed a detection zone 6. In this drawing, meat product 5 is shown to be delivered from an outlet of a preceding machine, which may be a meat grinder, into the spreader 2 of the apparatus. The meat passes over the spreader 2 which has the effect of spreading the meat across the conveyor 3 surface and reducing the thickness of the meat in a direction perpendicular to the direction of travel of the conveyor. The spread meat is then conveyed on the conveyor 3 towards the detector 4. Once the meat reaches the end of the conveyor 3, it falls from the conveyor 3 through the detection zone 6. Whilst falling, the meat is scanned by a beam 7 from the detector 4 for the presence of foreign matter. Information from the scans is interpreted by the detector 4 to detect the presence of foreign matter in the meat product 5. After falling through the detection zone 6, the meat and any detected foreign matter may be sorted, for example, by way of bins 8 and/or diverters or an additional conveyor as shown in Figure 1.

In alternative embodiments, the spread meat product falls directly from the spreader towards the detector(s), in which case no conveyor is required.

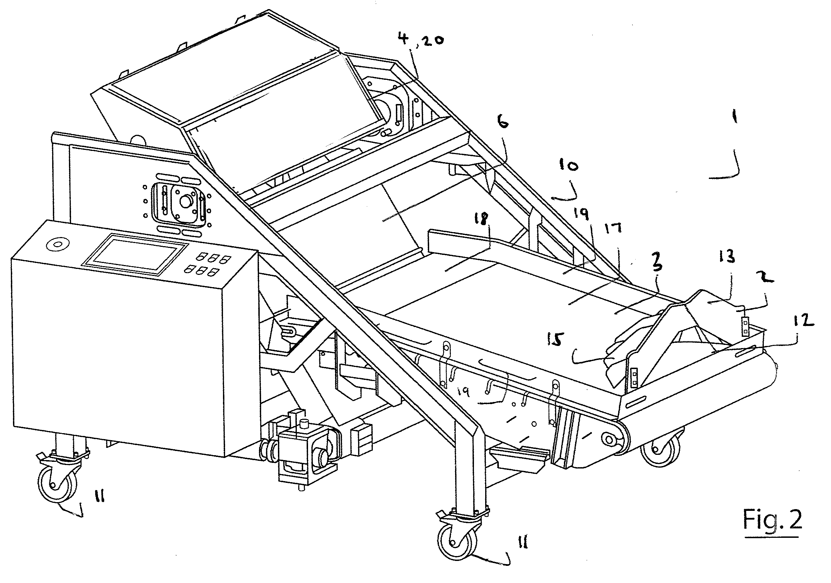

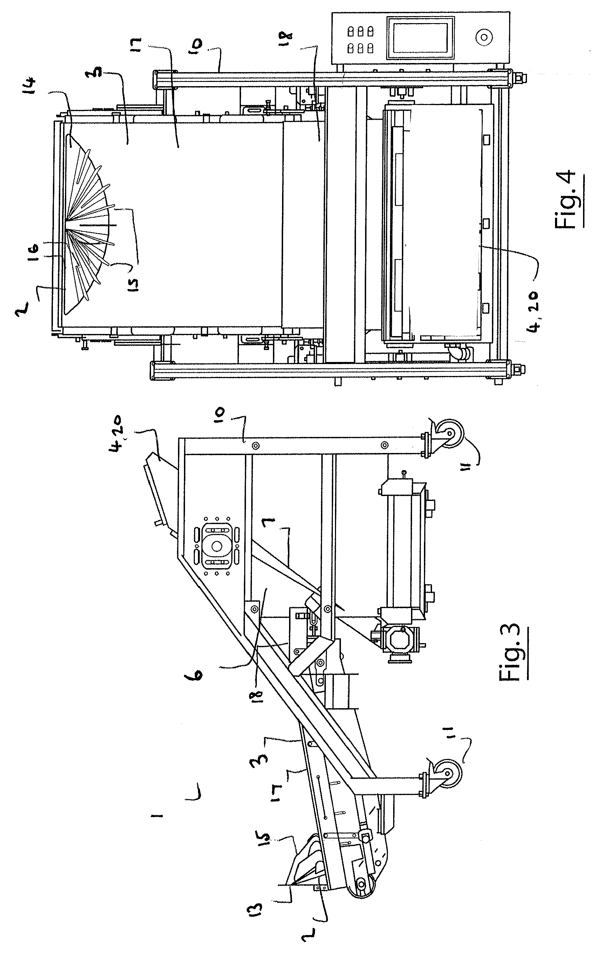

Figures 2 to 4 show a further embodiment of the apparatus 1 for detecting foreign matter according to the invention. The spreader 2, a conveyor 3, and a detector 4 are retained within a frame structure 10. The frame structure 10 is provided with wheels 11 for ease of movement of the apparatus.

The spreader 2 is positioned at one end of the conveyor 3 and is arranged to receive meat 5, for example from a meat grinder. The spreader is adapted for co-operation with an outlet of the meat grinder, as shown by recess 12 in a rear wall 13 of the spreader 2. The height of the spreader 2 and conveyor 3 may be fixed or adjustable to accommodate different types of grinders and other meat output apparatus. The spreader 2 consists of a substantially flat base surface 14 with a plurality of upright elongate separators 15. The separators 15 are substantially perpendicular to the flat base surface 14. The spreader 2 thus comprises a number of channels 16 defined by the separators 15. The separators provide channels whose width increases in a direction away from the rear wall 13. A flow or stream of meat is fed from the outlet onto the spreader 2 and through the channels 16. The separators 15 are fin-like structures which are shaped to spread or fan out received meat product 5 more evenly across the width of the conveyor 3. Spreading the meat in such a manner reduces clumping of the meat and reduces the thickness of meat distributed on the conveyor 3 surface. Reducing the thickness in this manner provides for more accurate detection of foreign matter at the detector 4. The separators extend radially from a region on the surface of the spreader 2 and are provided in two lengths, a first length and a second shorter length. The longer separators extend from the rear wall 13 of the spreader 2 and extend over the edge of the base surface 14 onto the conveyor 3 surface. The shorter separators extend from a region remote from the rear wall 13 but also extend over the edge of the base surface 14 onto the conveyor 3 surface. The separators 15 are arranged to alternate between the first length and the second length.

The conveyor 3 is arranged to convey meat from the spreader 2 towards the detector 4. The conveyor 3 consists of a first inclined portion 17 and a second substantially horizontal portion 18. The inclined portion provides that the apparatus may be used in conjunction which machines, for example meat grinders, having outlets positioned at a low height. The apparatus may therefore be retro-fitted to existing apparatus or production lines. The angle of incline of the inclined portion 17 is adjustable. In this embodiment of the invention, the inclined portion of the conveyor is inclined at an angle of about 10 degrees with respect to the horizontal.

The conveyor conveys the meat from the spreader, along the inclined portion 17 and onto the horizontal portion 18. The meat is then conveyed over the edge of the horizontal portion 18 and falls from the conveyor 3 into the detection zone 6. Whilst the meat falls in this region, it may be scanned by the detector 4. The horizontal portion 18 is arranged to provide greater control over the trajectory of the meat as it falls from the conveyor 3. In a further embodiment, the conveyor consists of a first inclined portion and a second declined portion. The declined portion provides an alternative trajectory to the meat falling from the conveyor.

The conveyor further comprises side guards 19 to prevent meat product from falling from the sides of the conveyor. The speed of the conveyor 3 is adjustable to optimise the conveyance of appropriate volume of meat product to the detector. The speed of the conveyor 3 is adjustable between about 0.1 m/s and about 3m/s.

The detector 4 is positioned about the end of the conveyor 3. The detector 4 comprises a spectrometer 20. A beam 7 from the spectrometer 20 is arranged to scan the meat product falling from the conveyor 3 through the detection zone 6. Information from the scan is analysed with software to detect the presence of foreign matter in the meat. In one embodiment of the invention, the wavelength of the beam 7 is in the visible or near infra-red, NIR, region. It will be appreciated that any detection method suitable for distinguishing foreign matter from meat may be employed in accordance with the invention, including the apparatus for detecting matter as disclosed in WO/2013/115650.

In a further embodiment, the detector is arranged to scan the meat product with respect to a reference surface such that the meat product falls between the detector and the reference surface. This provides for enhanced accuracy of detection. The reference surface may comprise a stainless steel surface which has the further advantage of being an accepted material for use within the food industry.

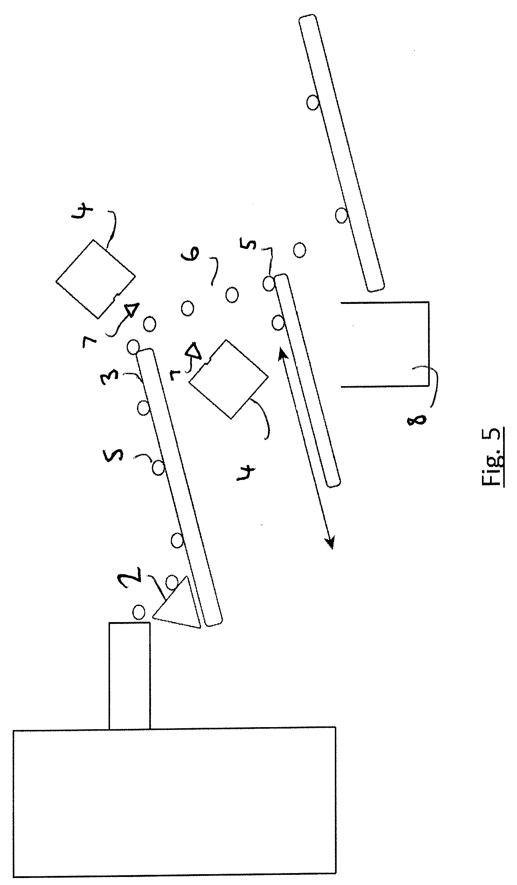

In the embodiment shown in Figure 5, multiple detectors are employed. The system comprises a first detector arranged to scan the meat product from a first direction and a second detector arranged to scan the meat product from a second direction, different from the first. More than two detectors may be provided in an alternative embodiment. Where dual detectors are employed, both sides of the stream may be scanned, where each detector is looking at different parts of the stream, such as front-and backside. In a further embodiment, a detector can consist of several units that each scan a part of the width of the spread meat product stream.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. An apparatus for detecting foreign matter in meat product comprising:
at least one detector (7) arranged to detect foreign matter in falling meat product; and
at least one spreader (2) for increasing the spread of meat product prior to its falling.

2. The apparatus of claim 1 further comprising a conveyor (3) across which the meat product is spread by the at least one spreader.

3. The apparatus of claim 2 wherein the conveyor (3) is a belt conveyor.

4. The apparatus of claim 2 or claim 3 wherein the conveyor (3) comprises at least one inclined portion.

5. The apparatus of any of claims 2 to 4 wherein the conveyor comprises at least two portions having different inclinations.

6. The apparatus of any preceding claim wherein the or each detector (7) is arranged to scan the falling meat product with respect to a reference surface, in use the meat product falling between the detector and the reference surface.

7. The apparatus of claim 6 wherein the reference surface comprises a stainless steel surface.

8. The apparatus of any preceding claim comprising a first detector arranged to scan the meat product from a first direction and a second detector arranged to scan the meat product from a second direction, different from the first.

9. The apparatus of any preceding claim wherein the at least one spreader (2) comprises a plurality of channels (16), such that meat received at the spreader is distributed between the channels.

10. The apparatus of claim 9 wherein the at least one spreader (2) comprises a plurality of elongate separators (15) defining the channels (16).

11. The apparatus of claim 10 wherein the plurality of elongate separators (15) extend radially from a region on the surface of the spreader.

12. The apparatus of claim 10 or 11 comprising elongate separators of a first length for providing a first separation of the meat and elongate separators of a second shorter length for providing a second separation of the meat.

13. The apparatus of claim 12 comprising alternating separators of the first length and the second length.

14. A method for detecting foreign matter in meat product comprising:
increasing the spread of the meat product; and dropping the spread meat product in front of at least one detector to detect foreign matter in the meat product when falling.
